# DEMANDE DE BREVET EUROPEEN

(11) **EP 1 243 282 A1**
(43) Date de publication de la demande: **25.09.2002**
(21) Numéro de dépôt: 02362004.0
(22) Date de dépôt: 20.03.2002
(51) Int. Cl.: A61M 16/00, A61M 16/12

(54) **Ventilateur pulmonaire**

(30) Priorité: 21.03.2001 FR 0103836
(71) Demandeur: Airox, 64008 Pau Cedex (FR)
(72) Inventeur: LeGrand, Michel, 64230 Lescar (FR); Andrieux, Claude, 64800 Baudreix (FR)
(74) Mandataire: Fantin, Laurent

(57) **Abrégé**

- L'objet de l'invention est un ventilateur pulmonaire comprenant un générateur de flux (12), avec en amont une prise d'air (14), équipée de préférence d'un filtre, et en aval, une sortie (16) du flux vers un patient, caractérisé en ce qu'il comprend une vanne de régulation (18) comportant deux clapets (20, 22), actionnés par le même actionneur (24), le premier clapet (20) autorisant ou non la circulation du flux produit par le générateur (12) vers le patient.

## Description

La présente invention se rapporte à un ventilateur pulmonaire, susceptible d'assurer différents modes de fonctionnement, par exemple de type volumétrique ou barométrique, en fonction de la ou des caractéristiques contrôlées du flux fourni.

Les ventilateurs pulmonaires existants comprennent un générateur de flux d'air comportant une entrée d'air munie généralement d'un filtre et une sortie dirigée vers le patient ainsi que des moyens de contrôle du flux d'air fourni, susceptibles d'ajuster différentes caractéristiques en fonction de l'état du patient.

Un premier principe de fonctionnement pour produire les différentes phases du cycle respiratoire, à savoir l'expiration et l'inspiration, consiste à activer le générateur de flux en fonction de la phase du cycle. Ainsi, ce dernier ne fonctionne qu'au moment de la phase d'inspiration du patient. Toutefois, cette solution ne donne pas pleinement satisfaction car le profil du flux fourni est imposé par les caractéristiques mécaniques du générateur et difficilement adaptable en fonction du besoin du patient.

Une autre solution est proposée par le document WO99/47197. Dans ce cas, le ventilateur pulmonaire comprend un générateur de flux avec une sortie reliée à une première extrémité d'un conduit patient, la seconde extrémité dudit conduit étant munie d'une canule permettant de contrôler le débit d'air insufflé au patient. Ce dispositif permet grâce au contrôle d'une fuite au niveau du conduit patient de gérer la pression délivrée au patient en faisant varier le débit de ladite fuite.

Un clapet disposé au niveau de la première extrémité du conduit du patient est également prévu pour gérer les phases d'inspiration et d'expiration du patient. Pour limiter la surpression au niveau du générateur de flux, au moment de la phase d'expiration, lorsque le clapet est fermé, le régime du générateur de flux est modifié.

Ce dispositif ne donne pas pleinement satisfaction pour les raisons suivantes.

Tout d'abord, ce dispositif ne permet pas de gérer un mode de fonctionnement volumétrique, permettant de contrôler le volume d'air insufflé au patient, en raison de la présence de la fuite au niveau du conduit du patient.

Par ailleurs, la nécessité de faire varier le régime du générateur de flux en fonction des phases respiratoires du patient induit nécessairement une surconsommation du générateur de flux.

Enfin, de manière générale, on constate que les ventilateurs pulmonaires existants sont relativement bruyants, et nécessitent une maintenance relativement importante et coûteuse.

Aussi, la présente invention vise à pallier les inconvénients des dispositifs de l'art antérieur en proposant un ventilateur pulmonaire de conception simple, polyvalent, susceptible d'assurer différents modes de fonctionnement, notamment volumétrique, avec des coûts de fonctionnement et d'entretien sensiblement inférieurs aux dispositifs existants.

A cet effet, l'invention a pour objet un ventilateur pulmonaire comprenant un générateur de flux, avec en amont une prise d'air, équipée de préférence d'un filtre, et en aval, une sortie du flux vers un patient, caractérisé en ce qu'il comprend une vanne de régulation comportant deux clapets, actionnés par le même actionneur, le premier clapet autorisant ou non la circulation du flux produit par le générateur vers le patient.

Cet agencement permet d'obtenir un régime sensiblement constant du générateur de flux, réduisant sa consommation énergétique.

Selon un mode de réalisation, le générateur de flux est disposé dans un caisson dans lequel est ménagée la prise d'air, l'entrée du générateur étant disposée à l'intérieur du caisson et aspirant l'air présent à l'intérieur, la sortie dudit générateur étant reliée à la vanne de régulation. De préférence, le caisson comprend à l'intérieur des moyens de circulation, sous forme de cloisons, de manière à assurer une meilleure circulation du flux autour du générateur. Cet agencement permet de refroidir efficacement le générateur de flux, et de la sorte augmenter sa durée de vie.

Avantageusement, le caisson comprend des moyens d'isolation phonique au niveau de ses parois de manière à isoler acoustiquement le générateur de flux.

Selon un autre caractéristique, le clapet réintroduit le flux en amont du générateur de flux lors de la phase expiratoire. Cet agencement permet de réduire l'encrassement du filtre d'aspiration.

Selon une autre caractéristique, le ventilateur pulmonaire comprend un circuit patient, relié à la sortie servant d'interface entre le patient et le ventilateur pulmonaire, et comportant un conduit principal reliant la sortie du ventilateur pulmonaire au masque ou à la canule de trachéotomie, ainsi qu'un clapet d'expiration.

Avantageusement, une électrovanne proportionnelle reliée au clapet d'expiration est également prévue pour assurer le contrôle du déclenchement de l'ouverture dudit clapet d'expiration.

D'autres caractéristiques et avantages ressortiront de la description qui va suivre de la carte de l'invention, description donnée à titre d'exemple uniquement et en regard des dessins annexés sur lesquels :
- la figure 1 est une vue schématique d'un ventilateur pulmonaire selon l'invention,
- la figure 2A est une vue schématique d'un premier mode de réalisation du circuit patient,
- la figure 2B est une vue schématique d'un autre mode de réalisation du circuit patient.

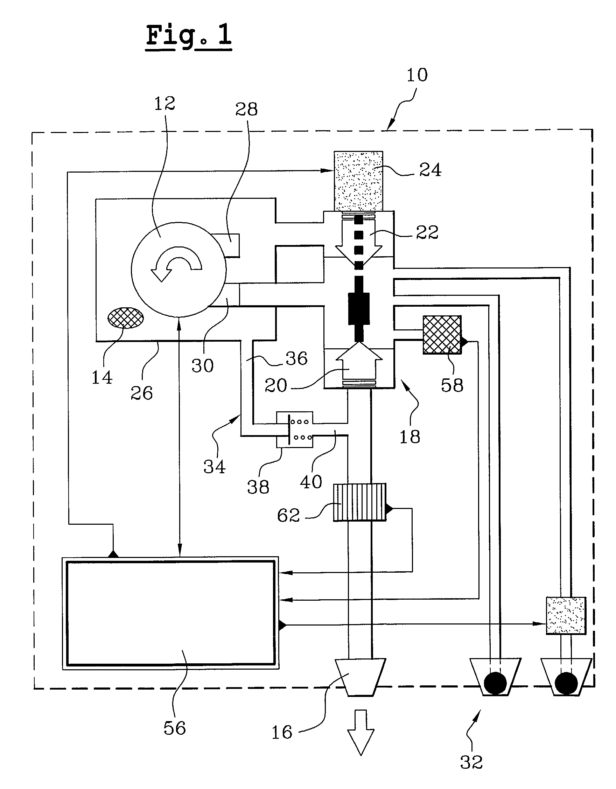

Sur la figure 1, un ventilateur pulmonaire 10 est illustré de façon schématique. Ce type d'appareil est susceptible d'assister le patient lors des phases respiratoires, inspiratoire et expiratoire, selon des modes de fonctionnement volumétriques et barométriques, en fonction de sa déficience respiratoire.

Il comprend un générateur de flux 12, avec en amont une prise d'air 14 équipée de préférence d'un filtre non représenté, et en aval, une sortie 16 du flux patient.

Selon l'invention, une vanne de régulation 18 est prévu entre le générateur de flux 12 et la sortie 16 de manière à soit réintroduire le flux en amont du générateur ou soit le mettre à l'échappement lors de la phase expiratoire du patient, le flux étant orienté vers le patient lors de la phase inspiratoire.

Grâce à cet agencement, le générateur est susceptible de fonctionner à régime constant, ce qui permet de limiter la consommation énergétique du générateur et d'obtenir un coût de fonctionnement réduit. Avantageusement, lorsqu'il y a recirculation du flux d'air en amont du générateur, le flux d'air généré n'est pas rejeté inutilement dans l'atmosphère, ce qui contribue à diminuer l'encrassement du filtre de la prise d'air 14.

Cette vanne de régulation 18 comprend deux clapets 20, 22, de préférence actionnés par le même actionneur 24, le premier clapet 20 autorisant ou non la circulation du flux produit par le générateur 12 vers le patient et le second clapet 22 autorisant ou non la réintroduction du flux produit par le générateur en amont de ce dernier.

Selon une caractéristique de l'invention, les clapets 20, 22 peuvent être fermés simultanément, mais l'ouverture de l'un d'eux n'est possible que lorsque l'autre est fermé. De préférence, l'actionneur 24 est susceptible de générer des lois d'ouverture différents pour les deux clapets 20, 22.

Dans tous les cas, le clapet 20 est en position fermée lors de la phase d'expiration, ce qui empêche la contamination du générateur de flux, de la vanne de régulation 18 ou de tout élément disposé en amont du clapet 20, par l'air expiré par le patient.

Avantageusement, les clapets, notamment le clapet 20, a un profil particulier permettant de régler le débit dirigé vers le patient en terme de niveau et de forme en fonction du temps. De préférence, le profil permet d'obtenir une fonction linéaire entre le déplacement du clapet et le débit.

L'actionneur 24 peut être de différents types, par exemple un moteur pas à pas ou un solénoïde.

Selon une autre caractéristique de l'invention, le générateur de flux 12 est disposé dans un caisson 26 dans lequel est ménagée la prise d'air 14, l'entrée 28 du générateur 12 étant disposée à l'intérieur du caisson 26 et aspirant l'air présent à l'intérieur dudit caisson 26, la sortie 30 dudit générateur étant reliée à la vanne de régulation 18 et le flux issu du clapet 22 de recirculation étant rejeté dans ledit caisson.

De façon préférentiellement, le caisson 26 comprend à l'intérieur des moyens de circulation, non représentés, sous forme de cloisons, de manière à assurer une meilleure circulation du flux autour du générateur. La circulation permanente d'air autour du générateur en phase inspiratoire comme expiratoire permet une évacuation continue des calories générées par le générateur et son moteur d'entraînement, tendant à améliorer leur durée de vie dépendante du niveau de sollicitation thermique.

Des moyens d'isolation phonique sont également prévus au niveau des parois du caisson de manière à isoler acoustiquement le générateur 12 de façon à rendre moins bruyant le ventilateur 10. Selon un mode de réalisation, les parois du caisson sont constituées ou bien tapissées à l'intérieur d'un matériau à amortissement phonique.

Selon une autre caractéristique du ventilateur de l'invention, ce dernier comprend une alimentation en oxygène 32 débouchant en amont des clapets 20 et 22 de la vanne de régulation 18. Ainsi, cette alimentation ne nécessite pas un moyen de pilotage distinct mais peut se faire sous forme de débit continu, la vanne de régulation 18 gérant les phases expiratoires et inspiratoires. Par ailleurs, lorsqu'il y a recirculation, contrairement aux dispositifs existants, l'oxygène n'est pas rejeté dans l'atmosphère pendant la phase expiratoire mais dans le caisson par le biais du clapet 22, ce qui réduit sensiblement la consommation d'oxygène, ce dernier étant accumulé dans le caisson 26.

Selon une autre caractéristique du ventilateur de l'invention, ce dernier comprend un circuit de respiration spontanée 34 dont l'entrée est disposée en amont du générateur 12 et la sortie en aval de la vanne de régulation 18. Ce circuit est prévu en cas de défection du générateur de flux 12 et/ou de la vanne de régulation 18. Selon un mode de réalisation, ce circuit 34 comprend une entrée 36 débouchant dans le caisson 26, un clapet 38 et une sortie 40 débouchant en aval de la vanne de régulation 18. Le clapet 38 est taré en fonction du seuil de déclenchement souhaité.

Grâce à cet agencement, la respiration ne peut s'effectuer qu'au travers du filtre de la prise d'air 14, ce qui contribue à améliorer la qualité de l'air, contrairement aux dispositifs de l'art antérieur qui prévoient cette entrée dans l'environnement mécanique et électronique très poussiéreux. Par ailleurs, l'air aspiré peut être enrichi en oxygène si le raccordement en oxygène est effectué.

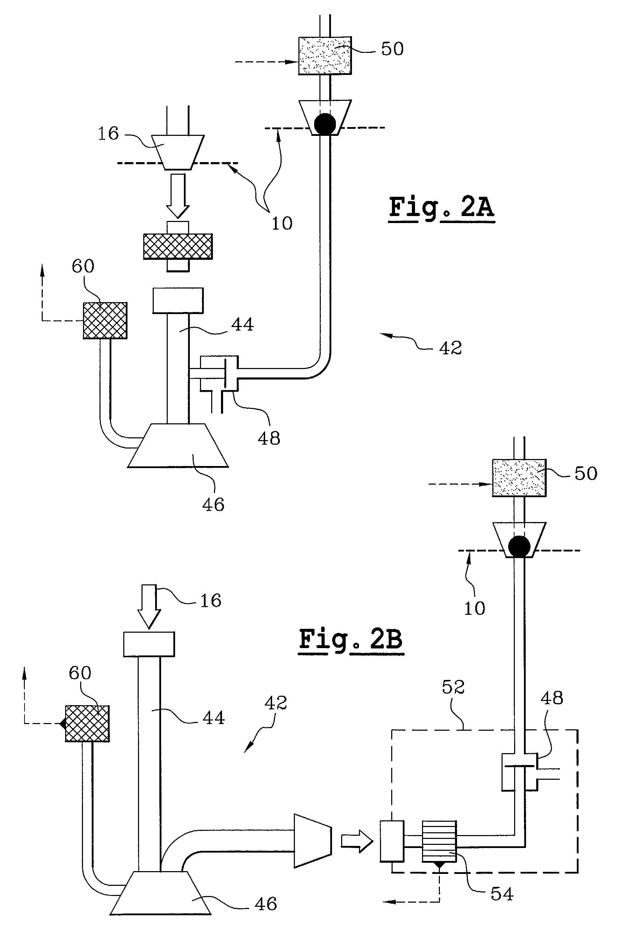

Un circuit patient 42, illustré par la figure 2A, est relié à la sortie 16 servant d'interface entre le patient et le ventilateur pulmonaire.

Il comprend un conduit principal 44 reliant la sortie 16 du ventilateur pulmonaire au masque 46 ou à la canule de trachéotomie, ainsi qu'un clapet d'expiration 48 relié audit conduit 44. On peut éventuellement prévoir un filtre entre la sortie 16 et le conduit principal.

Selon une autre caractéristique, des moyens de réglage du déclenchement de l'ouverture du clapet d'expiration 48 sont prévus de manière à autoriser une fuite du circuit patient et éventuellement pour maintenir un état de pression déterminée dans ce dernier lors de la phase d'expiration. Selon un mode de fonctionnement de ce clapet 48, le déclenchement de l'ouverture peut être fonction de la pression du flux au niveau de la vanne de régulation. A cet effet, le ventilateur pulmonaire comprend une électrovanne proportionnelle 50 reliant le clapet d'expiration 48 à la vanne de régulation 18 et contrôlant le déclenchement de l'ouverture dudit clapet.

La présence de ce clapet d'expiration 48 permet de supprimer la fuite nécessaire dans les dispositifs de l'art antérieur et de pouvoir obtenir un mode de fonctionnement barométrique et volumétrique.

Selon un autre mode de réalisation illustré par la figure 2B, le circuit patient 42 se présente sous la forme d'un circuit double branche connecté par un Y au masque, une des deux branches étant reliée à la sortie 16 du ventilateur pulmonaire alors que l'autre comportant à son extrémité distale un bloc d'expiration 52 permettant de surveiller le débit et le volume expiré par le patient. Ce bloc 52 comprend un capteur de débit 54 et un clapet d'expiration 48 relié à l'électrovanne proportionnelle 50.

En complément, de manière à piloter le générateur de flux 12, l'actionneur 24 de la vanne de régulation ainsi que l'électrovanne proportionnelle 50, le ventilateur pulmonaire comprend un calculateur 56 recevant d'une part les consignes de réglage des paramètres de fonctionnement et des alarmes déterminés au moyen d'un clavier, et d'autre part les signaux de capteurs, par exemple le capteur de débit expiré 54, un capteur de vitesse du générateur 12, un capteur de pression 58 du générateur, un capteur de pression patient 60 et un capteur de débit inspiré 62. En complément, on peut prévoir un capteur de pression venant mesurer la pression entre l'électrovanne 50 et le clapet 48 pour contrôler d'éventuelles défaillances du mécanisme et le rendre plus sûr.

Le principe de fonctionnement du ventilateur pulmonaire de l'invention est maintenant expliqué.

La gestion du flux d'air administré au patient tant au niveau du débit, de la pression et du volume est assuré par le calculateur 56 qui agit sur le générateur de flux 12, l'actionneur 24 de la vanne de régulation 18 et selon les modes de fonctionnement sur l'électrovanne proportionnelle 50.

A chaque mise en route du ventilateur pulmonaire, une phase de tests est effectuée, permettant d'une part, de vérifier l'intégrité des différents éléments et l'étanchéité des clapets, et d'autre part, de rechercher la position référence de l'actionneur 24 correspondant à l'état fermé des clapets 20 et 22.

Suite à cette phase de test, durant les premiers cycles de fonctionnement, le régime du générateur de flux est optimisé. Au départ, le générateur de flux fonctionne à un régime prédéterminé de manière à disposer des capacités de débit et de pression suffisantes. Ensuite, le régime est optimisé, le clapet patient 20 ayant de préférence une position médiane en position ouverte. Ainsi, si le clapet est trop ouvert, le régime du générateur est augmenté alors que si le clapet est faiblement ouvert, le régime du générateur est réduit. Suite à cette étape, le générateur fonctionne à régime constant, ce qui permet d'une part d'optimiser la consommation énergétique, et d'autre part de réduire les effets sonores liés aux variations importantes de régime.

Durant une phase d'inspiration, l'électrovanne proportionnelle 50 est ouverte, le clapet d'expiration 48 fermé et le clapet patient 20 de la vanne de régulation 18 est déplacé par l'actionneur 24 selon une loi de déplacement calculée dépendant de la forme de débit souhaitée en fonction du temps ou de la pression à atteindre, du volume total à administrer, du rapport de temps entre les phases inspiratoire et expiratoire et enfin de la fréquence de respiration souhaitée, la fixation de tout ou partie de ces paramètres dépendant du mode de ventilation désiré.

Comme pour le compresseur, la loi d'ouverture et de fermeture du clapet 20 pourra subir en temps réel de légères corrections en fonction de l'état des capteurs de pression et de débit de l'appareil.

La loi de commande du clapet 20 peut intégrer aussi des formes spécifiques relatives au début d'ouverture et à la fin de la fermeture afin de minimiser les effets sonores liés aux chocs entre le clapet et son poussoir à l'ouverture et entre le clapet et son siège à la fermeture.

Durant la phase d'expiration, le clapet 20 est fermé et le clapet 22 de la vanne de régulation est ouvert de manière à permettre la recirculation du flux.

La loi d'ouverture du clapet 22 peut être linéaire lorsque l'expiration du patient est libre de contrainte de pression ou contrôlée afin de maintenir un état de pression particulier dans la vanne de régulation lorsque l'expiration doit se faire en pression positive.

Comme pour le clapet 20, la loi de commande du clapet 22 peut intégrer des formes spécifiques relatives au début de l'ouverture et à la fin de la fermeture afin de minimiser les effets sonores.

Concernant l'électrovanne proportionnelle 50, cette dernière peut être fermée correspondant à une position ouverte du clapet 48 d'expiration lorsque l'expiration est libre, ou bien partiellement fermée afin de maintenir un niveau de pression positive dans le circuit du patient grâce à l'ouverture contrôlée du clapet d'expiration 48.

La présente description a était faite avec des moyens de recirculation permettant de réintroduire le flux d'air généré en amont du générateur, dans le caisson. En variante, comme nous l'avons déjà mentionné, le clapet 22 peut refouler le flux à l'échappement, dans l'atmosphère, sans que cela n'influe sur le mode de fonctionnement du ventilateur pour le patient.

Bien entendu, l'invention n'est évidemment pas limitée au mode de réalisation représenté et décrit ci-dessus, mais en couvre au contraire toutes les variantes, notamment en ce qui concerne les formes, dimensions et matériaux des différents conduits, du caisson, des capteurs de mesure, du type d'actionneur, des moyens de contrôle de la pression dans le circuit patient ainsi que sur le mode de ventilation administré au patient.

## Revendications

1. Ventilateur pulmonaire comprenant un générateur de flux (12), avec en amont une prise d'air (14), équipée de préférence d'un filtre, et en aval, une sortie (16) du flux vers un patient, **caractérisé en ce qu'**il comprend une vanne de régulation (18) comportant deux clapets (20, 22), actionnés par le même actionneur (24), le premier clapet (20) autorisant ou non la circulation du flux produit par le générateur (12) vers le patient.

2. Ventilateur pulmonaire selon la revendication 1, **caractérisé en ce que** le générateur de flux (12) est disposé dans un caisson (26) dans lequel est ménagée la prise d'air (14), l'entrée (28) du générateur (12) étant disposée à l'intérieur du caisson (26) et aspirant l'air présent à l'intérieur, la sortie (30) dudit générateur étant reliée à la vanne de régulation (18).

3. Ventilateur pulmonaire selon la revendication 2, **caractérisé en ce que** le caisson (26) comprend à l'intérieur des moyens de circulation, sous forme de cloisons, de manière à assurer une meilleure circulation du flux autour du générateur.

4. Ventilateur pulmonaire selon la revendication 2 ou 3, **caractérisé en ce que** le caisson (26) comprend des moyens d'isolation phonique au niveau de ses parois de manière à isoler acoustiquement le générateur de flux (12).

5. Ventilateur pulmonaire selon l'une des revendications 1 à 4, **caractérisé en ce que** le clapet (22) réintroduit le flux en amont du générateur de flux (12) lors de la phase expiratoire.

6. Ventilateur pulmonaire selon l'une quelconque des revendications 1 à 5, **caractérisé en ce qu'**il comprend un circuit de respiration spontanée (34) dont l'entrée est disposée en amont du générateur (12) et la sortie en aval de la vanne de régulation (18).

7. Ventilateur pulmonaire selon l'une quelconque des revendications 1 à 6, **caractérisé en ce qu'**il comprend une alimentation en oxygène (32) débouchant en amont des clapets (20, 22) de la vanne de régulation (18).

8. Ventilateur pulmonaire selon l'une quelconque des revendications précédentes, **caractérisé en ce qu'**il comprend un circuit patient (42), relié à la sortie (16) servant d'interface entre le patient et le ventilateur pulmonaire, et comportant un conduit principal (44) reliant la sortie (16) du ventilateur pulmonaire au masque (46) ou à la canule de trachéotomie, ainsi qu'un clapet d'expiration (48).

9. Ventilateur pulmonaire selon la revendication 8, **caractérisé en ce qu'**il comprend une électrovanne proportionnelle (50) reliée au clapet d'expiration (48) assurant le contrôle du déclenchement de l'ouverture dudit clapet d'expiration.

10. Ventilateur pulmonaire selon l'une quelconque des revendications 1 à 9, **caractérisé en ce qu'**il comprend un calculateur contrôlant le générateur de flux (12) et la vanne de régulation (18), renseigné par des capteurs.
